# EUROPEAN PATENT SPECIFICATION

(11) **EP 0 938 678 B1**
(45) Date of publication and mention of the grant of the patent: **03.09.2003**
(21) Application number: 97927218.4
(22) Date of filing: 23.06.1997
(51) Int. Cl.: G01N 33/68, G01N 33/53, C07K 14/47, G01N 33/96

(54) **METHOD AND KIT FOR THE DIAGNOSIS OF TROPONIN I**
VERFAHREN UND KIT ZUR DIAGNOSE VON TROPONIN I
PROCEDE ET TROUSSE SERVANT A EFFECTUER LE DIAGNOSTIC DE TROPONINE I

(30) Priority: 25.06.1996 US 670013
(43) Date of publication of application: 01.09.1999
(73) Proprietor: OY Aboatech AB, 20510 Turku (FI)
(72) Inventor: KATRUHKA, Alex G., Moscow, 113145 (RU); SEVERIN, Sergei, Moscow, 113149 (RU); BERSENIKOVA, Anastasia, Moscow, 103064 (RU); ESAKOVA, Tatiana, Moscow, 111401 (RU); PETTERSSON, Kim, FIN-20810 Turku (FI)
(74) Representative: Karvinen, Leena Maria
(86) International application number: FI9700399
(87) International publication number: WO97049994

(56) References cited:
- EP-A- 0 743 522
- GB-A- 2 275 774
- BIOCHEMISTRY AND MOLECULAR BIOLOGY INTERNATIONAL, Volume 36, No. 1, May 1995, ALEXEI G. KATRUKHA et al., "A New Method of Human Cardiac Troponin I and Troponin T Purification", pages 195-202.
- LABORATORY MEDICINE, Volume 23, No. 5, May 1992, HUGO A. KATUS et al., "Proteins of the Troponin Complex", pages 311-317.

## Description

### FIELD OF THE INVENTION

The object of the present invention is an improvement in an immunoassay method for assaying human cardiac and skeletal troponins I (TnI) in a sample taken from the blood stream of a patient.

A further object of the invention is a kit for use in an improved diagnostic immunoassay method for assaying troponin I in a sample from the blood stream of a patient.

A still further object of the invention is an improved troponin I standard preparation for use in the method and kit according to the invention.

### BACKGROUND OF THE INVENTION

Human cardiac troponin I (h.c. TnI) was recently suggested to be a specific and sensitive marker of myocardial cell death. It is released into the blood stream after myocardial damage, e.g. after acute myocardial infarction (AMI) (see e.g. Adams, J.E. et al., *Circulation* 1993; 88:101-106). Human skeletal troponin I (h.sk.TnI) has been suggested as a sensitive and specific marker of skeletal cell death.

TnI is the subunit of a troponin complex that plays an important role in the Ca²⁺-dependent regulation of vertebrate skeletal and cardiac muscle contraction. The troponin complex is located on the thin filament of the contractile apparatus and through its association with two other thin filament proteins, actin and tropomiosin, inhibits the actomyosin interaction at submicromolar Ca2+-concentrations, and stimulates interaction at micromolar and higher Ca²⁺-concentrations. The troponin complex contains three subunits - troponin C (TnC), which is the Ca²⁺-binding subunit; troponin I (TnI), which is the inhibitory subunit, and troponin T (TnT), which is the tropomiosin binding subunit. The interaction between the three subunits of the troponin complex is so strong that originally the purified troponin was thought to be a single protein.

The Ca²⁺-dependent regulation is initiated by conformational changes in TnC and subsequent changes in the interaction of TnC with TnI. TnC is composed of two globular domains connected by a central helix. Each domain contains two metal-binding sites. Two sites in the N-terminal domain (sites I and II) bind Ca²⁺ with low affinity, while sites in the C-terminal domain (sites III and IV) bind Ca²⁺ with high affinity. At submicromolar concentrations of Ca²⁺, the metal binding sites in the C-terminal domain of TnC are occupied with Mg²⁺, whereas the Ca²⁺-specific sites in the N-terminal domain are empty. In this case the N-terminal region of TnI is bound to the C-terminal domain of TnC, whereas the inhibitory C-terminal regions of the TnI molecule interact with actin and tropomiosin but not with TnC. When the Ca²⁺-concentration increases up to micromolar level, Ca²⁺ binds to the N-terminal, Ca²⁺-specific low affinity sites of TnC. The Ca²⁺-binding increases the affinity of this domain for the inhibitory and C-terminal regions of TnI, resulting in the release of those fragments from actin and tropomiosin and making strong contact with the extended central and N-terminal regions of TnC. This high-affinity binding of the components of the troponin complex results in important conformational changes of both molecules.

In human striated muscle, three forms of TnI were found: two for skeletal muscle (h.sk. TnI) and one for cardiac muscle (h.c. TnI). The three troponin forms have similar structures, but for human cardiac troponin I the existence of 33 extra amino acids in the N-terminal part of the molecule, as compared to the skeletal TnI isoform, was shown. This extra polypeptide and also some changes in the amino acids make it possible to differentiate human cardiac troponin I from the skeletal forms, for example by immunological methods. All existing diagnostic systems are based on immunological measurement of TnI in serum samples.

It has been shown that in the presence of micromolar and higher concentrations of Ca²⁺, a mixture of troponins I and C exists in the form of a complex with strong interaction between both molecules. However, when the concentration of Ca²⁺ in the solution decreases to submicromolar levels, Ca²⁺ is washed away from the metal binding centers of TnC. After the Ca²⁺ is removed, the interaction between the two proteins weakens and the conformation of TnI shows resemblance with the native (not complexed) protein.

The concentration of Ca²⁺ in human serum is high enough for the two-low affinity centers of TnC to bind Ca²⁺. It means that in the serum of patients with AMI or in patients with various skeletal muscle diseases, a major part of the TnI should be present in the form of a complex with TnC. Our experiments confirmed this hypothesis. We have shown that in the serum of AMI patients, the main part of TnI (50-90%) is presented in the form of a complex with TnC (and probably also with TnT). As mentioned above, the conformation of TnI in complex with TnC at high, i.e. micromolar or higher concentrations of Ca²⁺ differs from the conformation of free TnI, or of TnI in complex with TnC at submicromolar concentrations of Ca²⁺.

In common practice highly purified protein is used for the immunization of animals for the production of mono- or polyclonal antibodies. The conformation of purified TnI used for immunization and standard preparation is, however, different from that of TnI in complex with TnC. The change in conformation of the protein can decrease the affinity of the antibodies or make protein-antibody interaction impossible. In addition, due to the strong interaction between the two troponin molecules at micromolar and higher concentrations of Ca²⁺, TnC will cover part of the surface of the TnI molecule and block some of the epitopes for some antibodies generated by the immunization of animals with highly purified troponin I. As a result, immunoassays based on the use of such antibodies, such as the main part of the commercially available antibodies developed for the said purpose, measure only the free TnI and a part of the TnI in complex with TnC in case the affinity constant of the antibodies is changed due to TnI-TnC interaction; or they measure only the free TnI is case the epitopes of the antibody are completely covered by TnC. In any case the concentration of TnI assayed by such systems in AMI serum samples will be different (lower) than the real concentration of this protein.

Thus there is a need for a method which would improve assays using such antibodies. Such a method would be useful not only in cases of diagnostic systems designed for assaying the cardiac form of TnI in serum but also in immunoassays developed for measurement of skeletal forms of TnI for diagnosis of skeletal muscle tissue necrosis.

In common practice, purified antigen is usually used for the preparation of calibrators or standard preparations for the immunoassays. In case of TnI, the purification is usually a complicated process that takes several days and includes several steps of column chromatography (affinity, ion exchange, etc.). It is a well known fact that TnI is highly susceptible to proteolysis. During long-term purification, the troponin I molecule can be partially cleaved by proteases and thus some part of the epitopes for some antibodies can be lost. In addition, all existing methods for TnI purification include stages in which highly concentrated (6-8M) urea solutions are used to dissociate the components of the troponin complex. Such rigid treatment can lead to irreversible changes in the conformation of some parts of the TnI molecule. Consequently the conformation of the TnI molecules in highly purified preparations can be different from that of the native protein. In addition, long-term contact of the protein with urea at high concentrations can result in a partial carbamylation of the TnI molecule. Thus the immunological activity of highly purified TnI can be different from that of native TnI.

### SUMMARY OF THE INVENTION

The object of the invention is to provide an improved immunoassay diagnostic method for assaying troponin I in a sample from the blood stream of a patient, comprising bringing the sample in contact with a Ca²⁺-binding agent, and determining the troponin I concentration in the sample using a standard, the standard comprising a complete troponin complex.

The amount of Ca²⁺-binding agent used is such as to be sufficient to decrease the Ca²⁺ -concentration in the sample to submicromolar level, thus decreasing the TnI-TnC interaction.

A further object of the invention is a kit for use in a method for improving the sensitivity of a diagnostic immunoassay method for assaying troponin I in a sample from the blood stream of a patient, the kit containing a mono- or polyclonal antibody to troponin I, a Ca²⁺-binding agent, a detectable label and a standard in the form of a complete troponin complex.

A still further object of the invention is the use of a complete native troponin complex, isolated and purified from muscle tissue, for the preparation of a TnI-calibrator or standard. Such a standard preparation is used in the assay method and the kit according to the invention.

### DETAILED DESCRIPTION OF THE INVENTION

The method according to the invention is carried out as an immunoassay method using mono- or polyclonal antibodies to TnI, that is antibodies either to human cardiac or skeletal troponin I depending on the specific troponin antigen to be assayed.

The immunoassay method can be any one of the known immunoassay methods suitable for the purpose, such as an enzyme immunoassay, radioimmunoassay, chemiluminescence immunoassay or fluoroimmunoassay method, etc. Such immunoassay systems are well known to a person skilled in the art and also commercially available.

According to one embodiment of the invention, the method is performed as a sandwich immunoassay method, using pairs of preferably monoclonal anti TnI antibodies, one antibody functioning as a "capture" antibody immobilized on a solid phase, and the second antibody carrying the label and functioning as the "detection" antibody.

According to the invention, such a diagnostic assay method can be improved by bringing the sample to be assayed into contact with an agent or reactant capable of binding or complexing with Ca²⁺. According to the invention, such a Ca²⁺-binding agent can be any substance which, on the one hand, has an affinity constant to Ca²⁺ sufficient to reduce the Ca²⁺ level to submicromolar, and, on the other hand, does not negatively interfere with the assay method.

The Ca²⁺-binding agent is preferably used in the form of a solution, preferably an aqueous solution, or a buffer solution, compatible with the assay.

A preferred group of Ca²⁺-binding agents are the metal chelate forming agents which bind metals, including Ca²⁺, in aqueous solutions. The group of metal chelate forming agents is well known to the person skilled in the art and includes such substances as polyoxycarboxylic acids, polyamines, ethylenediamine tetraacetic acid (EDTA), nitrilotriacetic acid (NTA) and ethyleneglycol-O,O'-bis(2-aminoethyl)-N,N,N',N'-tetraacetic acid (EGTA), and similar agents. Preferred substances are EDTA and EGTA in aqueous solution.

According to the invention, the Ca²⁺-binding agent in the form of a solution is brought into contact with the sample to be assayed. This can take place by adding the Ca²⁺-binding solution directly to the sample or, in a sandwich immunoassay method, to the buffer used in the assay during incubation with the blood sample.

The Ca²⁺-binding agent is used in an amount sufficient to decrease the troponin I - troponin C interaction by binding enough Ca²⁺ in order to reduce the level of Ca²⁺ in the assay medium to submicromolar level. The decrease of interaction results in better exposure of TnI which then can be assayed.

The standard for use in the method of the invention comprises the complete native troponin complex, containing TnI, TnT and TnC in equimolar concentration, which can be isolated from muscle tissue, such as human cardiac tissue, using for example a method slighthy modified from the method described in Katrukha et al., *"Biochemistry and Molecular Biology International"* vol. 36, No. 1, 1995, p. 195-202. By using the whole complex, rather than purified TnI, one can avoid problems for example associated with changes in some epitopes on the troponin I which can take place for example during purification. We have also shown that the stability of TnI in the form of the native troponin complex is considerably higher compared to that of purified TnI, commonly used as a standard.

The present invention also contemplates a kit for use in an immunoassay method as defined above. Such a kit would include at least one mono- or polyclonal antibody to TnI, a Ca²⁺-binding agent, preferably in the form of an aqueous solution, a detectable label, and a complete troponin complex as the standard. The label is attached to one of the immunoreactive components in order to provide a means for indicating the degree of immunological interaction and thus to allow for the determination of the TnI level in the sample. Consequently the label is of a type which can be detected by conventional methods for detection, such as using methods based on absorbance, luminescence, fluorescence or radioactivity. The detected signal can then be compared to standard curves. The standard curves are constructed by using in the standard preparation known amounts of the full troponin complex containing the three subunits described above, which has been derived directly from muscle tissue. Such a preparation has the advantage that it has better immunological properties than a preparation containing purified TnI. The troponin complex in the standard can be combined with a Ca²⁺ binding agent, such as EDTA, added for example in a buffer, prior to measurement.

According to a preferred embodiment, the kit contains two monoclonal antibodies to TnI, to allow for a sandwich type of immunoassay. The first or capture antibody can in such a case be pre-attached to a solid phase, such as the wall of a micro-plate well. The second or detection antibody, which carries the label, can be provided in a suitable buffer solution. The Ca²⁺-binding agent can be provided separately in an aqueous or buffer solution, or incorporated in the solution of the second antibody.

It is also possible to formulate the kit in the form of a well known immunochromatography strip. Such a strip can easily be used with whole blood, for example in order to diagnose AMI at an early stage, e.g. already in an ambulance in the case of emergency.

According to a preferred embodiment, we have used a two-step time resolved fluoroimmunoassay method, LANFIA, lanthanide fluoroimmunoassay. In this method a first biotinylated TnI antibody (capture antibody) is coated onto a solid phase, for example the surface of a streptavidin-coated micro plate well. After washing, the coated surface is then incubated in a second step with a mixture of the sample and a second labeled, e.g. Eu-chelate labeled antibody (detection antibody) in a buffer. A solution of the Ca²⁺-binding agent can be added either to the sample or to the detection antibody solution. After washing and adding a LANFIA enhancement solution, the fluorescence signal (counts per second) is measured in a conventional manner. A description of the use of time-resolved fluorometry in immunoassay is to be found for example in *"Alternative Immunoassays"*, ed. by Collins, W.P., John Wiley & Sons Ltd., 1985, Chapter 12.

### DETAILED DESCRIPTION OF THE DRAWING

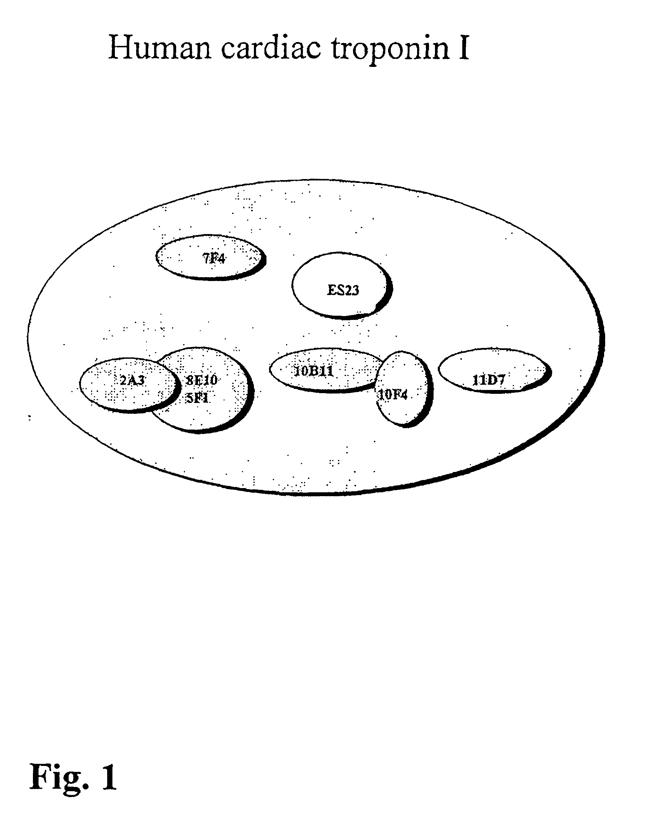

In the drawing, Fig. 1 shows the troponin I epitope map. Five antigenic domains can be discerned for eight anti-troponin I monoclonal antibodies, tested in our experiments. Three monoclonal antibodies (7F4, 11D7 and ES23) have unique epitopes; 8E10 and 5F1 have one and the same epitope; the epitopes of 10F4 and 10B11; and 8E10, 5F1 and 2A3 are overlapping.

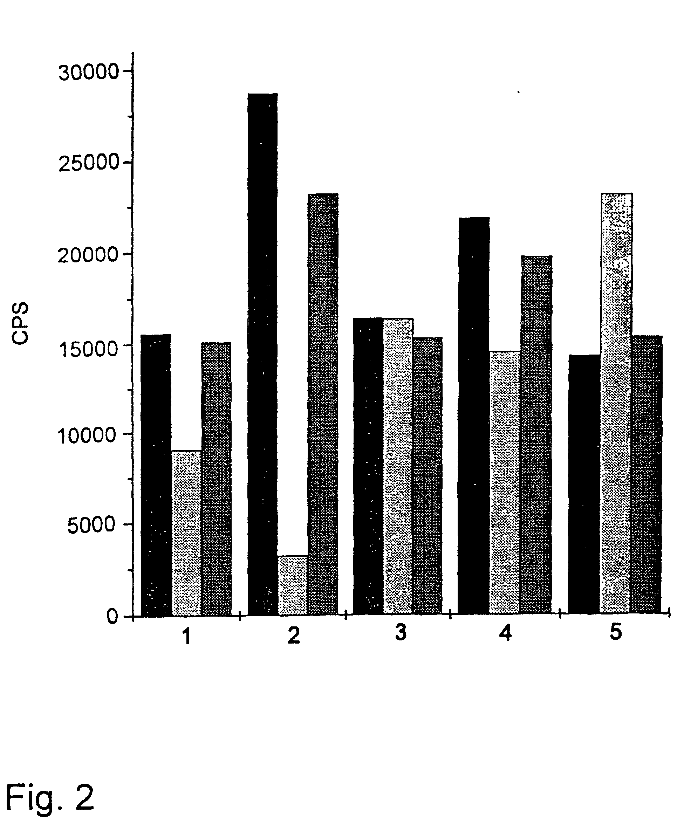

Figure 2 shows the effect of troponin C on the immunological activity of troponin I for several pairs of antibodies (1-5 respectively):

| N | Biotinylated Ab | Eu-labeled Ab |
|---|---|---|
| 1 | 10F4 | 8E10 |
| 2 | 7F4 | ES23 |
| 3 | 7F4 | 2A3 |
| 4 | 10B11 | 2A3 |
| 5 | 7F4 | 10B11 |

The left hand column (black) shows the signal obtained while assaying a troponin I solution (30 ng/ml TnI in normal human serum), the middle column (light gray) the effect of adding five molar excess of troponin C, and the right hand column (dark grey) the effect of EDTA as a Ca²⁺- binding reagent, added to the incubation mixture of TnI and TnC. Almost all assays (except # 3) are effected to a different degree by adding TnC to a troponin I standard preparation. EDTA, added to an incubation mixture, restores the immunological activity of troponin I. Only for one antibody, ES23, the epitope remains partially covered (changed) by troponin C even in the presence of EDTA.

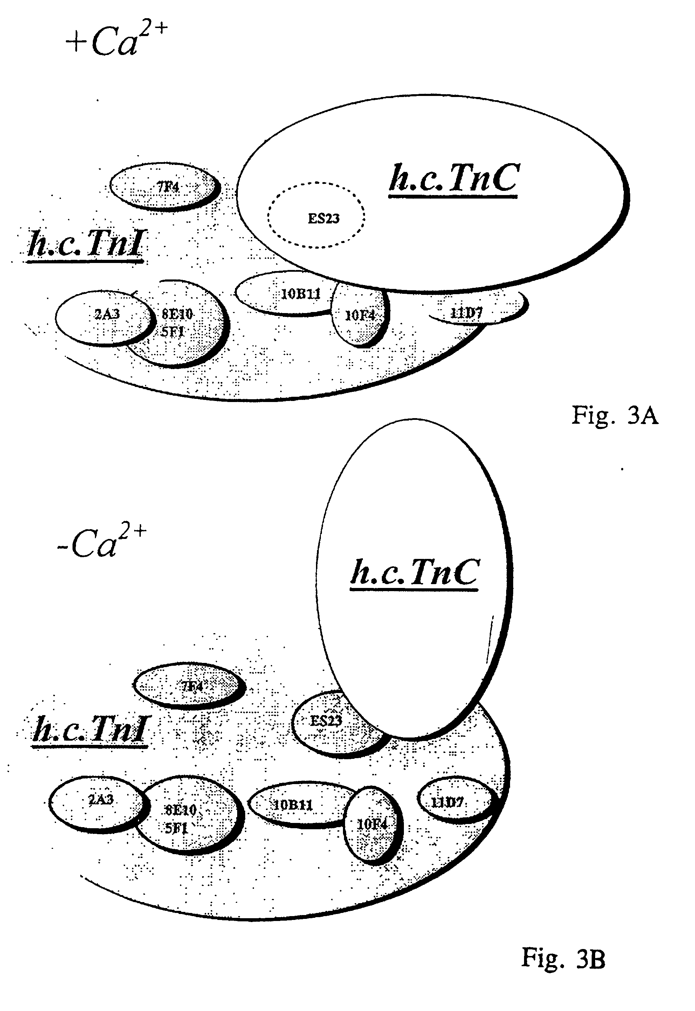

Figure 3 shows the epitope map for tested antibodies in the presence of troponin C. The epitopes of 10B11, 10F4, 11D7 and ES23 antibodies are changed or covered with troponin C in the presence of Ca²⁺ (Fig. 3A). In case the concentration of Ca²⁺ is decreased to a submicromolar level (-Ca²⁺), the epitopes of all antibodies (except the epitope of ES23 antibody) are not covered or changed any more (Fig. 3B).

Figure 4 shows the results of testing three serum samples (1, 2, 3) from AMI patients in three different assay systems (A, B, C).

| | | |
|---|---|---|
| N | Biotinylated Ab | Eu-labeled Ab |
| A | 7F4 | ES23 |
| B | 10F4 | 8E10 |
| C | 2A3 | 10B11 |

The left hand column gives the signal from a serum sample without Ca²⁺-binding agent, and the right hand column a similar sample but with added EDTA. The increase in the signal level after the addition of Ca²⁺-binding agent to the sample is evident for the first two systems and is less evident in the case of the last system, suggesting that the epitopes of 2A3 and 10B11 antibodies are not strongly changed by troponin I - troponin C interaction.

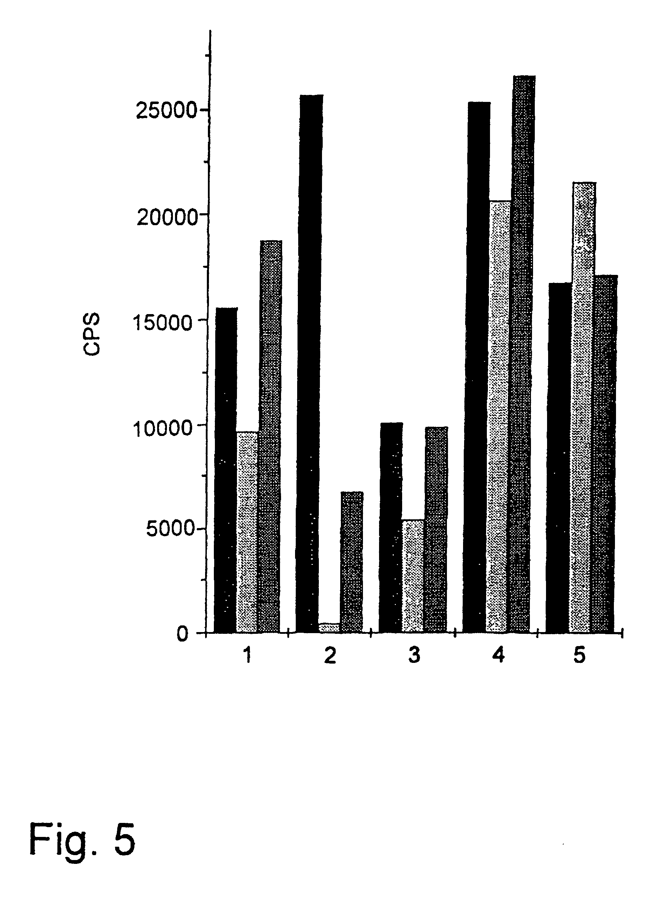

Figure 5 shows the comparison of immunological activities of highly purified troponin I, dissolved in normal human serum in a concentration of 30 ng/ml and troponin I in the form of the native complex (purified in mild conditions) in the same concentration.

| N | Biotinylated Ab | Eu-labeled Ab |
|---|---|---|
| 1 | 10F4 | 8E10 |
| 2 | 7F4 | ES23 |
| 3 | 10F4 | 10B11 |
| 4 | 8E10 | 7F4 |
| 5 | 7F4 | 10B11 |

The black column shows the signal, obtained while assaying a troponin I solution, the light gray column shows the signal level when troponin complex in the presence of Ca²⁺ was assayed, and the third column (dark grey) indicates the signal received when troponin complex in the presence of EDTA was assayed. For three tested pairs (# 3,4,5) troponin I in the form of the native troponin complex in the presence of EDTA has the same immunological activity as the highly purified troponin I. For 10F4-biot - 8E10-Eu antibodies, troponin I in complexed form has better activity than the purified troponin I. This fact confirms that during long term purification some epitopes of troponin I can change. From this point of view it is better to use troponin I in the form of the native complex for troponin I standard preparation. Only for one, ES23 antibody, the epitope remains partially covered (changed) by troponin C even in the presence of EDTA, as was the case when troponin C was added to troponin I solution.

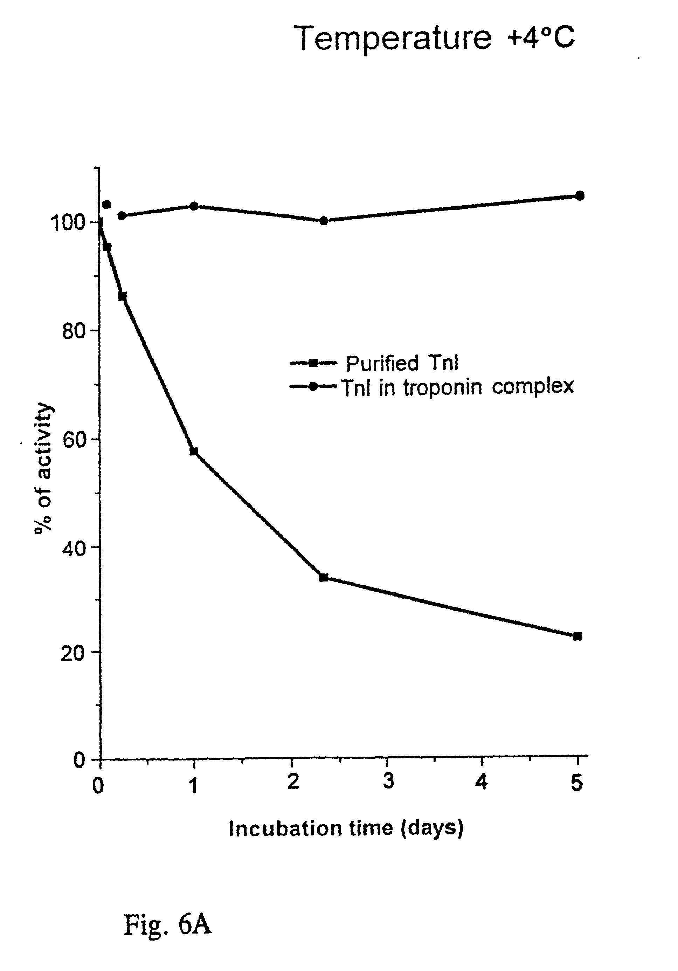

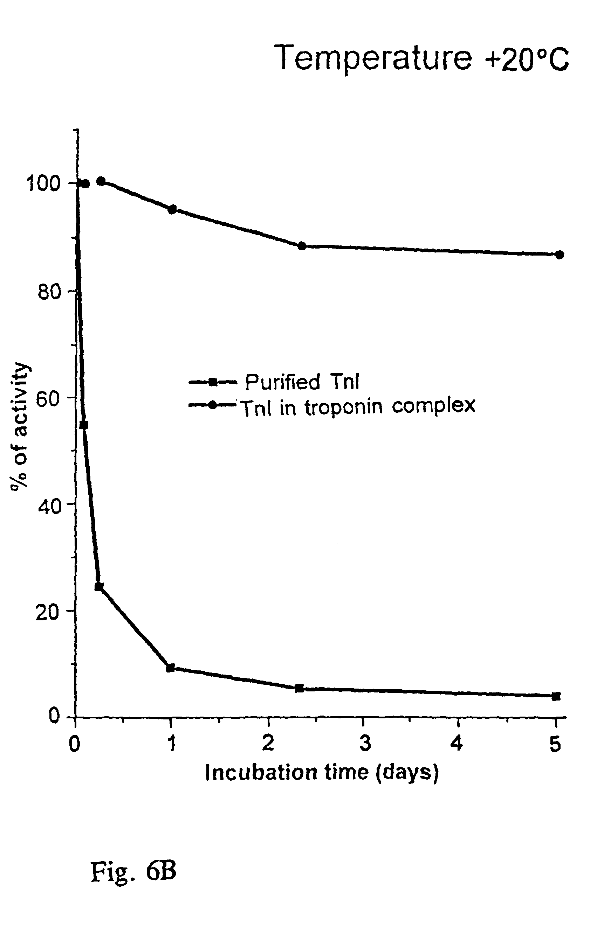

Figure 6A and B show the stability of purified TnI and TnI in the troponin complex incubated for one week at 4 and 20 °C, respectively. The tests were carried out using human cardiac troponin complex (HyTest, Turku) in a 10F4-Biot- 7F4-Eu assay. The complex was purified in mild conditions and contains all the components in equimolar proportion. The signal obtained in the assay with Tnl in the form of the complex was higher than the signal obtained in assay with purified Tnl. It confirms that during purification of Tnl, the protein can change the native conformation. The figures 6A and 6B indicate that the stability of Tnl in the native troponin complex is significantly higher than the stability of purified Tnl protein.

### EXAMPLES

### Troponin I purification

The troponin I that we used for mouse immunization, hybridoma testing and for standard preparation was prepared as is described in *"Biochemistry and Molecular Biology International"* vol. 36, No 1, 1995, p. 195-202.

### Troponin complex preparation

For the troponin complex preparation we have used a method slightly modified from the method described in *"Biochemistry and Molecular Biology International"*, vol.36, No 1, 1995, p.195-202.

10 grams of muscle tissue were homogenized at top speed in 100 ml of buffer 1 containing 20 mM Tris/HCl, pH 7.5, 0.1 mM phenylmethylsulfonyl fluoride (PMSF), 5 mM CaCl₂. The homogenate was centrifuged for 10 min. at 10,000 g and the pellet was homogenated for a second time in the same volume of the same buffer, containing 1% of Triton X-100. After centrifugation the pellet was homogenated in 100 ml of 150 mM potassium phosphate buffer (buffer 2) pH 6.5, containing 0.3 M KC1, 0.2 mM ATP, 1 mM PMSF. After centrifugation for 30 min. at 10000 g the pellet was suspended in 100 ml of cold 25 mM Tris/HCl buffer, pH 7.5 containing 0.7 M Lid, 5 mM CaCl₂, PMSF (buffer 3). The suspension was stirred for 30 min. at 0°C and then centrifuged for 1 hour at 10000 g. The supernatant was filtrated through paper filters and then loaded on an affinity column containing immobilized anti-troponin I monoclonal antibody (clone C5, antibodies of this clone crossreact with the skeletal and cardiac forms of troponin I wherefore this column can be used for the preparation of skeletal and cardiac forms of troponin). The affinity column was preequilibrated with buffer 3. The column was washed with several volumes of buffer 3 and troponin was eluted with 0.1 M glycine buffer pH 2.0, containing 5 mM CaCl₂. Then the protein was dialyzed against buffer 3 overnight. The yield is usually 12-15 mg of troponin from 10 g of muscle tissue. According to the SDS-gel electrophoresis, the troponin was sligthly contaminated by unidentified proteins. The concentration of troponin I and troponin T was determined by scanning gels with troponin complex and troponin I and troponin T standard preparations.

### Monoclonal antibody preparation

BALB/c mice were immunized with purified troponin I using a standard protocol: Briefly, mice were injected intraperitoneally on day 1 with 100 µg of human cardiac troponin I in complete Freund's adjuvant. On days 31 and 61, the mice were boosted intraperitoneally with 100 µg of troponin I in incomplete Freund's adjuvant. The final boosts were administered on days 91 and 93 with 50 µg of the antigen in phosphate-buffered saline, pH 7.4, injected both intraperitoneally and intravenously. On day 96, the mice were killed, their spleens were removed, and splenocytes were isolated for fusion. Splenocytes were fused to a nonsecretor cell line sp2/0 and plated into Dulbec-co's Modified Eagle's Medium containing hypoxanth, aminopterin, and thymidine (HAT) with 15% of fetal bovine serum.

Wells exhibiting hybridoma growth in HAT medium were screened for the production of anti-troponin I antibodies. For this purpose, hybridoma culture media were incubated for 30 minutes at 37°C in micro-ELISA plates coated with troponin I (300 ng/well) and after washing incubated with HRP-labeled goat anti-mouse IgG antibodies. After washing, HRP activity was determined by using o-phenylenediamine/hydrogen peroxide as a substrate and 1 M sulfuric acid as a stop reagent and 30 minutes for color development. Positive hybridomas were retested for antibody specificity by using micro-ELISA wells coated with the skeletal form of troponin I.

Hybridomas selected on the basis of specificity were cloned by two rounds of limiting dilution into aminopterin-free (HT) medium. Stable hybridoma clones were cultured as ascite tumors in BALB/c mice. Monoclonal antibody specificity was checked once more using h.c.TnI and h.sk.TnI coated micro-ELISA plates and different dilution of ascites fluid.

Monoclonal antibodies were purified from ascites fluids with Protein A-Sepharose affinity chromatography (Pharmacia). Antibody concentrations were determined by Lowry method using mouse IgG (Calbiochem) as a standard.

The specificity of monoclonal antibodies was confirmed by Western blotting. Purified monoclonal antibodies were incubated (3 µg/ml, 1 hour, 37°C) with Western blot membranes, containing purified proteins - h.c.TnI and h.sk.Tnl transferred from a SDS-PAGE gel (7.5-15 % gradient gel). After washing and one hour of incubation with HRP-labeled goat anti-mouse antibodies at 37°C, protein bands were made visible after incubation with 4-chloro-1-naphthol/hydrogen peroxide substrate. All antibodies described here were h.c.TnI specific without crossreaction with h.sk.TnI.

### Antibody biotinylation

For antibody biotinylation we have used biotinamidocaproate N-hydroxysuccinimide ester. Briefly the biotinylation reagent was dissolved in dimethyl sulfoxide in a concentration of 10 mg/ml. The antibody solution (3 mg/ml) was prepared in 0.1 M borate buffer pH 8.8. The biotinylation reagent was added to the antibody solution at a ratio 200 µg of ester per milligram of antibody, mixed well and incubated at room temperature for 4 hours. Then 17 µl of 1M NH₄Cl per 200 µg of biotin ester were added and after 10 min. of incubation at room temperature, the buffer was changed using NAP™-5 and NAP™-10 columns (Pharmacia, Uppsala, Sweden) preequilibrated with 50 mM Tris/HCI buffer pH 7.75 containing 0.9% of NaCl and 0.05% of NaN₃.

### Antibody Eu-labeling

Stable fluorescent chelates of EU used to label the detection antibodies were obtained from Wallac Oy (Turku, Finland). We used an Eu chelate of 4-[2-(4-isothiocyanatophenyl)ethynyl]-2,6-bis{[N,N-bis(carboxymethyl)-amino]methyl}pyridine. Labeling of monoclonal antibodies with the Eu-chelate was performed overnight at + 4°C with a 200-fold molar excess of the chelate in 50 mM sodium carbonate buffer, pH 9.8 The labeled antibodies were separated from the excess of free chelate by gelfiltration on a Superdex™ 200 HR 10/30 column (Pharmacia, Uppsala, Sweden) The column was preequilibrated with 50 mM Tris/HCl buffer pH 7.75 containing 0.9% of NaCl and 0.05% of NaN₃. The labeling degree of the pooled antibody fraction was determined against an Eu-calibrator.

### Pre-incubation of streptavidin-coated plates with biotinylated anti-TnI antibodies.

Biotinylated antibodies were incubated in the wells of a streptavidin-coated micro-well plate for 30 mins at room temperature using a concentration of 400 ng of antibody per well in 0.2 ml of DELFIA® buffer, with gentle shaking. DELFlA assay buffer contains, per liter, 50 mM Tris/HCl, pH 7.75, 9 g of NaCl, 5 g of BSA, 0.5 g of bovine serum γ-globulin, 0.1 g of Tween^{R} 40, 20 µM DTPA, 0.5 g of NaN₃ and 20 mg of cherry red.

After incubation, the plates were washed two times with DELFIA® washing solution.

### Incubation of labeled antibodies with serum samples in the pre-absorbed plates

In the second step, AMI serum samples were incubated with Eu-labeled antibodies (200 ng/well) and with EDTA in 5 mM concentration for 30 min at room temperature while gently shaking. 0.025 ml of serum and 0.1 ml of antibody solution in Delfia buffer per well was used. The EDTA-solution can be added either directly to the serum sample or to the labeled antibody solution.

After incubation, the plate was washed 6 times with DEL-FIA® washing solution and thereafter 0.2 ml of LANFIA enhancement solution per well was added. After incubation for 3 min with gently shaking, the signal was measured.

Instead of using a EDTA as solution in water, a solution in DELFIA® buffer (pH adjusted to 7.7-7.8) can be used. In place of EDTA, EGTA (ethyleneglycol-O,O'-bis(2-aminoethyl)-N,N,N',N'-tetraacetic acid) in solution with water or buffer can be used in the same concentration as EDTA.

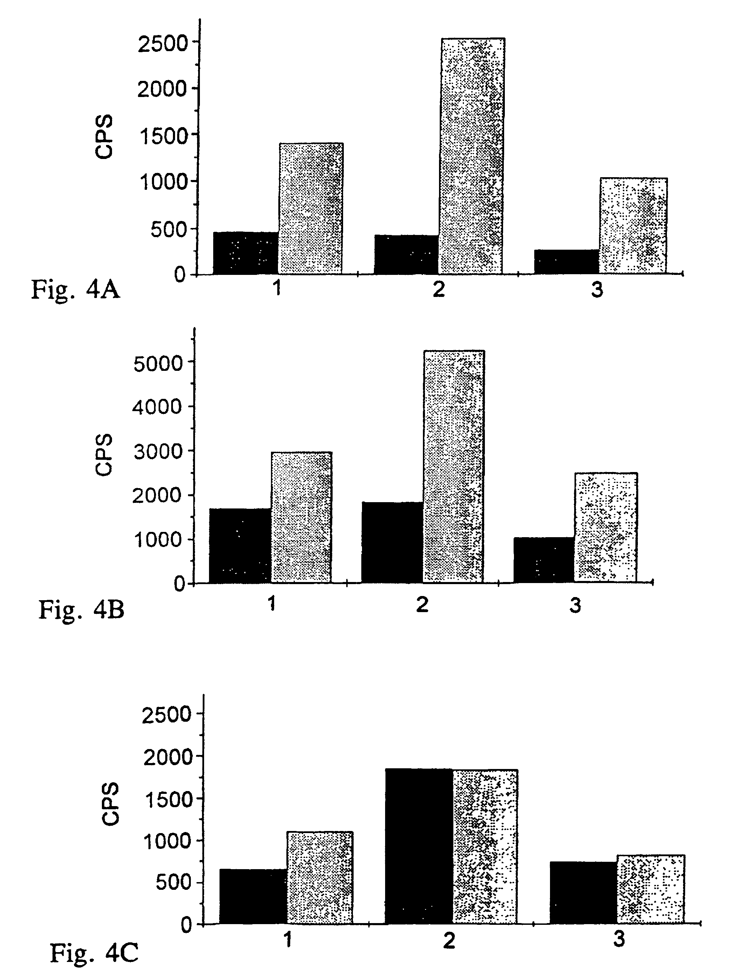

The above system was also used for obtaining the results shown in the Figs 2, 4 and 5, by using instead of the serum sample, troponin I solution in normal human serum with or without troponin C, made for example according to the method of Katrukha et al., *"Biochemistry and Molecular Biology International"* vol. 36, No 1, 1995, p. 195-202.

### Epitope mapping

In our experiments we have used eight monoclonal antibodies specific to the cardiac form of human troponin I without crossreaction with skeletal forms of troponin I. All possible combinations of those antibodies for sandwich fluoro immunoassay were tested. The first monoclonal antibody was biotinylated and immobilized on microtiter strip wells, the second one was labelled with europium chelate as is described above. All combinations were tested for reactivity with highly purified human cardiac troponin I. The Fig. 1 shows the troponin I epitope chart based on the checked board experiments and determination of crossreactivities.

Troponin C while binding to troponin I in the presence of Ca²⁺ covers part of the troponin I surface, thus changing the epitopes of some anti-troponin I antibodies, developed after immunization of the animal by purified troponin I. We have checked in sandwich immunoassay the interaction of different pairs of antibodies with purified troponin I and with troponin I in the presence of 5 molar excess of troponin C (with and without Ca²⁺). Figure 2 shows that for some pairs of antibodies the addition of troponin C to troponin I standard decreases the signal level. Addition of 5 mM EDTA restores the immunological activity of troponin I by decreasing troponin I - troponin C interaction.

## Claims

1. Method of improving an immunoassay diagnostic method for assaying troponin I (TnI) in a sample from the blood stream of a patient comprising
- bringing the sample in contact with a Ca²⁺-binding agent, and
- determining the troponin I content of the sample using a standard, which standard is a preparation of a complete native troponin complex containing TnI, TnT and TnC in equimolar concentration.

2. The method according to Claim 1, wherein the Ca²⁺-binding agent is provided in the form of an aqueous solution.

3. The method according to Claim 2, wherein the Ca²⁺-binding agent is a metal chelate forming agent.

4. The method accoding to Claim 2, wherein the Ca²⁺-binding agent is a metal chelate forming agent selected from EDTA and EGTA.

5. The method according to Claim 1, wherein the amount of Ca²⁺-binding agent added to the sample is sufficient to reduce the concentration of Ca²⁺ to a submicromolar level.

6. The method according to Claim 1, wherein the diagnostic method is a fluoroimmunoassay method.

7. The method according to Claim 1, wherein the diagnostic method is a sandwich immunoassay, using a first capture anti TnI antibody and a second detection anti TnI antibody.

8. The method according to Claim 7, wherein the Ca²⁺binding agent is added to a solution of the second antibody.

9. The method according to Claim 7, wherein the Ca²⁺binding agent is added to the blood sample.

10. The method according to claim 1, wherein the human cardiac troponin I content is determined.

11. Troponin I standard and calibrator preparation comprising a complete native troponin complex containing TnI, TnT and TnC in equimolar concentration, isolated from cardiac or skeletal muscle tissue in mild conditions, in normal human serum.

12. Method for preparing a troponin I standard or calibrator comprising
- isolating and purifying from cardiac or skeletal muscle tissue a complete native troponin complex containing TnI, TnT and TnC in equimolar concentration, in mild conditions, and
- adding the complex obtained to normal human serum, and
- optionally adding a Ca²⁺-binding agent.

13. Kit for improving an immunoassay diagnostic method for assaying troponin I in a sample from the blood stream of a patient, the kit containing
- a mono- or polyclonal antibody to troponin I,
- a Ca²⁺-binding agent,
- a detectable label, and
- a standard comprising a complete native troponin complex.

14. The kit according to Claim 13, wherein the Ca²⁺-binding agent is a metal chelate forming agent in the form of an aqueous solution.

15. The kit according to Claim 14, wherein the metal chelate forming agent is selected from the group consisting of EDTA and EGTA.

16. The kit according to Claim 13, wherein the detectable label is a fluorescent label.

17. The kit according to claim 13, containing
- a first monoclonal capture antibody to human cardiac troponin I, optionally immobilized onto a solid phase,
- a second monoclonal detection antibody to human cardiac troponin I, and
- a fluorescent label on the second antibody.

18. The kit according to claim 13, containing
- a first antibody immobilized onto a solid phase using streptavidin-biotin interaction,
- a Eu-chelate labeled second antibody, and
- an aqueous or buffered solution of EDTA.

## Patentansprüche

1. Verfahren zur Verbesserung eines diagnostischen Immuntestverfahrens zum Testen von Troponin I (Tnl) in einer Probe aus dem Blutstrom eines Patienten, wobei das Verfahren die folgenden Schritte umfasst:
- Inkontaktbringen der Probe mit einem Ca²⁺-Bindemittel, und
- Bestimmen des Gehalts an Troponin I in der Probe unter Verwendung eines Standards, wobei der Standard eine Zubereitung eines kompletten, nativen Troponinkomplexes ist, der Tnl, TnT und TnC in äquimolarer Konzentration enthält.

2. Verfahren nach Anspruch 1, wobei das Ca²⁺-Bindemittel in Form einer wässrigen Lösung bereitgestellt wird.

3. Verfahren nach Anspruch 2, wobei das Ca²⁺-Bindemittel ein ein Metallchelat bildendes Mittel ist.

4. Verfahren nach Anspruch 2, wobei das Ca²⁺-Bindemittel ein ein Metallchelat bildendes Mittel ist, das ausgewählt ist aus EDTA und EGTA.

5. Verfahren nach Anspruch 1, wobei die Menge des Ca²⁺-Bindemittels, das der Probe zugesetzt wird, ausreicht, um die Ca²⁺-Konzentration auf eine submikromolaren Spiegel zu verringern.

6. Verfahren nach Anspruch 1, wobei das diagnostische Verfahren ein Fluorimmuntestverfahren ist.

7. Verfahren nach Anspruch 1, wobei das diagnostische Verfahren ein Sandwich-Immuntest unter Verwendung eines ersten Fänger (capture)-anti-Tnl-Antikörpers und eines zweiten Nachweis-anti-Tnl-Antikörpers ist.

8. Verfahren nach Anspruch 7, wobei das Ca²⁺-Bindemittel der Lösung des zweiten Antikörpers zugesetzt wird.

9. Verfahren nach Anspruch 7, wobei das Ca²⁺-Bindemittel der Blutprobe zugesetzt wird.

10. Verfahren nach Anspruch 1, wobei der Gehalt an menschlichem Herztroponin I bestimmt wird.

11. Troponin I-Standard- und -Eichmaterialzubereitung, umfassend einen kompletten nativen Troponin-Komplex, der Tnl, TnT und TnC in äquimolarer Konzentration enthält, isoliert aus Herzmuskel- oder Skelettmuskelgewebe unter milden Bedingungen in normalem menschlichem Serum.

12. Verfahren zur Herstellung eines Troponin I-Standards oder -Eichmaterials, wobei das Verfahren die folgenden Schritte umfasst:
- Isolieren und Reinigen eines kompletten nativen Troponin-Komplexes, der Tnl, TnT und TnC in äquimolarer Konzentration enthält, von Herzmuskeloder Skelettmuskelgewebe unter milden Bedingungen, und
- Hinzufügen des erhaltenen Komplexes zu normalem menschlichem Serum, und
- gegebenenfalls Hinzugeben eines Ca²⁺-Bindemittels.

13. Kit zur Verbesserung eines diagnostischen Immuntestverfahrens zum Testen von Troponin I in einer Probe aus dem Blutstrom eines Patienten, wobei der Kit die folgenden Bestandteile enthält:
- einen mono- oder polyclonalen Antikörper gegen Troponin I,
- ein Ca²⁺-Bindemittel,
- einen nachweisbaren Marker, und
- einen Standard, der einen kompletten nativen Troponin-Komplex umfasst.

14. Kit nach Anspruch 13, wobei das Ca²⁺-Bindemittel ein Metallchelat bildendes Mittel in Form einer wässrigen Lösung ist.

15. Kit nach Anspruch 14, wobei das Metallchelat bildende Mittel ausgewählt ist aus der Gruppe bestehend aus EDTA und EGTA.

16. Kit nach Anspruch 13, wobei der nachweisbare Marker ein Fluoreszenzmarker ist.

17. Kit nach Anspruch 13, enthaltend
- einen ersten monoclonalen Fängerantikörper gegen menschliches Herz-Troponin I, gegebenenfalls auf einer Festphase immobilisiert,
- einen zweiten monoclonalen Nachweisantikörper gegen menschliches Herztroponin I, und
- einen Fluoreszenzmarker auf dem zweiten Antikörper.

18. Kit nach Anspruch 13, enthaltend
- einen ersten Antikörper, der unter Verwendung einer Streptavidin-Biotin-Wechselwirkung auf einer Festphase immobilisiert wurde,
- einen mit Eu-Chelat markierten zweiten Antikörper, und
- eine wässrige oder gepufferte EDTA-Lösung.

## Revendications

1. Procédé pour améliorer une méthode de diagnostic par immunodosage destinée au dosage de la troponine I (TnI) dans un échantillon provenant du courant sanguin d'un patient, comprenant les étapes consistant à
- mettre l'échantillon en contact avec un agent liant le Ca²⁺, et
- déterminer la teneur en troponine I de l'échantillon en utilisant un étalon, lequel étalon est une préparation d'un complexe complet de troponines natives contenant de la TnI, de la TnT et de la TnC en concentration équimolaire.

2. Procédé selon la revendication 1, dans lequel l'agent liant le Ca²⁺ est proposé sous forme de solution aqueuse.

3. Procédé selon la revendication 2, dans lequel l'agent liant le Ca²⁺ est un agent formant un chélate métallique.

4. Procédé selon la revendication 2, dans lequel l'agent liant le Ca²⁺ est un agent formant un chélate métallique choisi parmi l'EDTA et l'EGTA.

5. Procédé selon la revendication 1, dans lequel la quantité d'agent liant le Ca²⁺ ajoutée dans l'échantillon est suffisante pour réduire la concentration en Ca²⁺ à un niveau submicromolaire.

6. Procédé selon la revendication 1, dans lequel la méthode de diagnostic est une méthode de dosage immunofluorimétrique.

7. Procédé selon la revendication 1, dans lequel la méthode de diagnostic est une méthode d'immunodosage en sandwich, utilisant un premier anticorps anti-TnI de capture et un second anticorps anti-TnI de détection.

8. Procédé selon la revendication 7, dans lequel l'agent liant le Ca²⁺ est ajouté dans une solution du second anticorps.

9. Procédé selon la revendication 7, dans lequel l'agent liant le Ca²⁺ est ajouté dans l'échantillon de sang.

10. Procédé selon la revendication 1, dans lequel la teneur en troponine I cardiaque humaine est déterminée.

11. Préparation d'un étalon et calibrateur de la troponine I comprenant un complexe complet de troponines natives contenant de la TnI, de la TnT et de la TnC en concentration équimolaire, isolé à partir de tissu musculaire cardiaque ou squelettique dans des conditions douces, dans du sérum humain normal.

12. Procédé de préparation d'un étalon ou calibrateur de la troponine I comprenant les étapes consistant à
- isoler et purifier à partir de tissu musculaire cardiaque ou squelettique un complexe complet de troponines natives contenant de la TnI, de la TnT et de la TnC en concentration équimolaire, dans des conditions douces, et
- ajouter le complexe obtenu dans du sérum humain normal, et
- ajouter facultativement un agent liant le Ca²⁺.

13. Trousse pour améliorer une méthode de diagnostic par immunodosage destinée au dosage de la troponine I dans un échantillon provenant du courant sanguin d'un patient, la trousse comprenant
- un anticorps mono- ou polyclonal dirigé contre la troponine I,
- un agent liant le Ca²⁺,
- un marqueur détectable, et
- un étalon comprenant un complexe complet de troponines natives.

14. Trousse selon la revendication 13, dans laquelle l'agent liant le Ca²⁺ est un agent formant un chélate métallique sous forme de solution aqueuse.

15. Trousse selon la revendication 14, dans laquelle l'agent formant un chélate métallique est choisi dans le groupe constitué par l'EDTA et l'EGTA.

16. Trousse selon la revendication 13, dans laquelle le marqueur détectable est un marqueur fluorescent.

17. Trousse selon la revendication 13, contenant
- un premier anticorps de capture monoclonal dirigé contre la troponine I cardiaque humaine, facultativement immobilisé sur une phase solide,
- un second anticorps de détection monoclonal dirigé contre la troponine I cardiaque humaine, et
- un marqueur fluorescent sur le second anticorps.

18. Trousse selon la revendication 13, contenant
- un premier anticorps immobilisé sur une phase solide en utilisant l'interaction streptavidine-biotine,
- un second anticorps marqué avec un chélate d'Eu, et
- une solution aqueuse ou tamponnée d'EDTA.
